# EUROPEAN PATENT SPECIFICATION

(11) **EP 4 551 498 B1**
(45) Date of publication and mention of the grant of the patent: **25.03.2026**
(21) Application number: 23741316.6
(22) Date of filing: 07.07.2023
(51) Int. Cl.: F16L 11/20, B67D 1/04, B67D 1/08, B67D 1/12, G01N 33/24, G01N 33/14

(54) **BEVERAGE DISPENSE SYSTEM**
GETRÄNKEAUSGABESYSTEM
SYSTÈME DE DISTRIBUTION DE BOISSON

(30) Priority: 07.07.2022 GB 202209992
(43) Date of publication of application: 14.05.2025
(73) Proprietor: Heineken UK Limited, Edinburgh, Lothian EH12 9JZ (GB)
(72) Inventor: BRIANT, Stuart, Edinburgh, Lothian EH12 9JZ (GB)
(74) Representative: Mewburn Ellis LLP
(86) International application number: PCT/EP2023/068936
(87) International publication number: WO 2024/008963

(56) References cited:
- EP-A1- 3 162 757
- WO-A1-2017/194929
- AU-B2- 503 415
- DE-A1- 102006 026 025
- DE-U1- 202010 004 970
- DE-U1- 202013 002 350
- GB-A- 2 327 748
- US-A- 5 287 913
- US-A1- 2011 309 109

## Description

### Technical Field

The present disclosure relates to a beverage dispense system for dispensing a beverage, such as beer or cider.

### Background

Beverage dispensing systems are known for dispensing draught beverages. In such systems, the beverage is typically stored at a beverage supply (e.g. a keg) that is remote from a dispensing site (e.g. a tap) and is transferred on demand to the dispensing site. Such transfer of beverage is often provided by a pressurised gas system.

A common issue with such beverage dispensing systems (especially of the type where the dispensing site is remote from the supply) is microorganism growth. Such growth can spoil beverage within the various beverage lines, components and any kegs of the system.

One way of inhibiting such growth is to maintain the beverage at a temperature that is below that at which such growth occurs. To maintain the beverage at such a temperature as it travels from the beverage source to the dispensing site it is known to provide a cooler. In some systems, the beverage line passes through the cooler itself. Alternatively or additionally, a cooling line may be provided that extends along the beverage line to cool the beverage line.

One component of a beverage system that can be particularly susceptible to microorganism growth is a foam on beer (fob) detector. A fob detector detects the presence of foam in a beverage line, which his indicative of a keg being empty (or nearly empty) and requiring replacement. A traditional fob detector is in the form of a receptacle having a float that blocks an outlet of the receptacle when foam is present therein.

Using a cooler to cool the beverage line can help to reduce microorganism growth, but can require significant energy to do so. Even then such cooling does not always ensure the prevention of microorganism growth across the entire system.

The present disclosure has been devised in light of the above considerations.

WO2017194929A1 proposes a connector for use in a beverage dispense system, the connector defining a conduit for connecting a beverage line to a beverage supply, the connector comprises an electrical sensor (e.g. a capacitive sensor) for measuring an electrical parameter of a beverage within the connector conduit, the electrical parameter and/or changes in the electrical parameter can be used to detect bubbles in the beverage and/or identify the type/brand of beverage.

### Summary

The invention is defined by the appended set of claims.

In a first aspect according to the invention, there is provided a beverage dispense system, the system comprising:
a beverage flow path along which beverage flows in use from a beverage supply to a dispense site;
a coolant flow path along which coolant flows from a cooler;
a connector at an upstream end of the beverage flow path configured for releasable connection to the beverage supply, the connector comprising a beverage passage along which a portion of the beverage flow path extends and a coolant passage along which a portion of the coolant flow path extends;
a sensor comprising two spaced probes arranged so as to be in contact with beverage flowing through the beverage passage in use to measure a parameter of the beverage; and
a flexible tube-in-tube conduit extending from the connector, the conduit comprising an inner beverage tube extending within an outer coolant tube, the beverage tube connected to the beverage passage so as to define a portion of the beverage flow path and the coolant tube connected to the coolant passage so as to define a portion of the coolant flow path.

The provision of a connector that includes a coolant passage ensures that beverage passing through the connector (within the beverage passage) can be cooled by the coolant. In this way, the temperature of beverage leaving the beverage supply (e.g. a keg) can be maintained at a level that prevents (or at least reduces) microorganism growth. By providing the sensor probes within this portion of the system (i.e. within the beverage passage of the connector) microorganism growth is also prevented in the vicinity of the probes (such growth could otherwise foul the probes and cause inaccurate measurements, as well as spreading elsewhere in the system). The provision of a tube-in-tube conduit (i.e. with an outer coolant tube) connected to the connector further reduces microorganism growth in this region of the beverage system, so as to further facilitate maintenance of the temperature of the beverage at a desirable temperature (i.e. below that at which microorganism growth occurs).

For the avoidance of doubt, unless explicitly specified, the terms "downstream" and "upstream" are used in the context of the direction of flow of beverage in normal use (i.e. in a direction away from the beverage supply).

Optional features of the first aspect will now be set out. These are applicable singly or in any combination with any aspect.

The connector may comprise a beverage inlet configured for releasable engagement with the beverage supply, for example, with a keg or a keg coupler. The connector may comprise a beverage outlet connected to the beverage tube. The beverage passage may extend from the beverage inlet to the beverage outlet.

The connector may comprise a coolant inlet and a coolant outlet. The coolant passage may extend from the coolant inlet to the coolant outlet. As may be appreciated, the coolant inlet or the coolant outlet may be connected to the coolant tube, depending on the direction of coolant flow along the coolant flow path.

A coolant line may extend from the cooler to the connector. The coolant line may be a coolant return line (for returning coolant from the connector to the cooler). The coolant return line may be connected to the coolant outlet of the connector.

Alternatively, the coolant line may be a coolant supply line (i.e. the cooler may be configured to supply coolant along the coolant line). The coolant supply line may be connected to the coolant inlet of the connector.

When the coolant line is a coolant supply line, coolant may flow through the connector in the same direction as the beverage (i.e. may flow from the connector to the conduit). In either case, the coolant line may be external to the tube-in-tube conduit (henceforth referred to as the "conduit") but may extend alongside the conduit.

A portion of the coolant passage of the connector may circumferentially surround (e.g. may be concentrically arranged about) the beverage passage of the connector. The beverage passage may comprise an upstream portion adjacent the beverage inlet and a downstream portion adjacent the beverage outlet. The downstream portion may be circumferentially surrounded by the coolant passage (e.g. may be arranged concentrically within the coolant passage). The beverage outlet and one of the coolant inlet and outlet may be co-axial (i.e. may share a central axis).

The upstream and downstream portions of the beverage passage may be arranged on an angle to one another. Thus, the beverage passage may comprise a bend between the upstream and downstream portions. The upstream and downstream portions of the beverage passage may be substantially perpendicular to one another.

The coolant passage may comprise first and second portions. The second portion of the coolant passage may circumferentially surround (e.g. be concentric about) the second portion of the beverage passage. The first and second portions of the coolant passage may be angled with respect to one another. The coolant passage may comprise a bend between the first and second portions. The first and second portions of the coolant passage may be substantially perpendicular to one another.

The upstream portion of the beverage passage may be arranged so as to be substantially perpendicular to the first portion of the coolant passage. For example, in an in-use orientation, the upstream portion of the beverage passage may extend substantially vertically and the first portion of the coolant passage may extend horizontally.

The first portion of the coolant passage may be arranged on an opposite side of the bend (between the upstream and downstream portions of the beverage passage) to the upstream portion of the beverage passage. For example, in an use orientation, the upstream portion of beverage passage may extend substantially vertically, and the first portion of the coolant passage may be disposed above (e.g. directly) the upstream portion of the beverage passage.

The probes of the sensor may be elongate. In other embodiments each probe may be in the form of e.g. an annular element surrounding beverage flow path extending through the beverage passage.

The probes may have substantially the same length. The probes may extend through a wall portion of the connector, which may be a portion of an external wall of the connector. The wall portion may be adjacent to the beverage passage (e.g. the upstream portion of the beverage passage). The wall portion may separate the beverage passage from the external environment. That is, an internal surface of the wall portion may partly define the beverage passage and an external surface of the wall portion may be an external surface of the connector. The wall portion may partly define the upstream portion of the beverage passage. The wall portion may be on an opposite side of the connector to the beverage outlet. In this way, the sensor may be positioned away from coolant/beverage in the conduit (which could otherwise affect operation of the sensor).

Each probe may be provided with a sealing element providing a seal between the probe and the wall portion. For example, the sealing element may be in the form of an annular sealing ring extending circumferentially about the probe. For example, the sealing ring may be in the form of a compressible washer that is received about the probe and within an opening in the wall portion through which the probe extends (providing a watertight seal between the probe and the wall portion).

At least one of the probes may extend so as to be substantially parallel to at least a portion of the beverage passage (i.e. parallel to the direction in which beverage flows through the passage in use). The at least one probe may extend so as to be substantially parallel to the downstream portion of the beverage passage. The at least one probe may extend in a direction that is transverse to the upstream portion of the beverage passage. The at least one probe may extend within the bend between the upstream and downstream portions of the beverage passage.

The probes may be substantially parallel to one another. The probes may include an upstream probe and a downstream probe. The upstream probe may be disposed closer to the beverage inlet than the downstream probe. The probes may be spaced in a direction of extension of the beverage passage (e.g. in a direction of extension of the upstream portion of the beverage passage). Thus, the probes may be spaced in a direction of beverage flow along the beverage passage.

The sensor may be a bubble sensor configured to detect the presence of bubbles in the beverage passage. This may be indicative of the presence (or imminent presence) of fob in the beverage passage, which may be indicative of the associated beverage supply being empty. The sensor may be configured to detect conductivity or capacitance of the beverage in the beverage passage.

The sensor may be mounted to the connector (e.g. to the external wall of the connector). The sensor may be releasably mounted to the connector (e.g. by mechanical fastener, such as a screw, or by snap engagement).

The system may comprise a valve configured to obstruct the flow of beverage along the beverage flow path. The valve may be a solenoid valve. The valve may be configured to obstruct the beverage flow path without obstructing flow of coolant along the coolant flow path. In this way, cooling can be maintained regardless of whether the beverage flow is obstructed.

The valve may be operatively connected to the sensor (e.g. by wired or wireless connection) for receiving a signal from the sensor. The valve may be operatively connected to the sensor directly, or indirectly (e.g. via controller). For example, the system may comprise a controller. The controller may be configured to receive measurements from the sensor and communicate a signal (e.g. a "close" signal) to the valve in response to the measurements. Components of the sensor and the controller may be provided on a printed circuit board (PCB).

The valve may be configured to close the beverage flow path in response to a signal generated by the sensor (e.g. communicated by the controller). For example, as noted above the sensor may be a bubble sensor. The sensor may be configured to generate a signal indicate of the presence of and/or the level of bubbles in the beverage. In this respect, the valve may be configured to close the beverage flow path in response to a measurement indicative of bubbles in the beverage (for example, due to a change in measured resistance, capacitance, conductivity, etc.).

The system may comprise a reset actuator (e.g. a push button). The valve may be configured to open in response to a signal generated by the reset actuator (e.g. when the reset actuator is actuated, such as when it is depressed). The reset actuator may, for example, be operatively connected to the controller (which may provide the signal to the valve). The reset actuator may be mounted to the PCB.

The reset actuator may comprise an indicator. For example, in the case of a push button, the button may comprise a light (such as an LED). The indicator may be configured to indicate a state of the system (e.g. a state of the valve). The indicator may be configured to indicate the state of the beverage passage (or the beverage supply). For example, the indicator may indicate when the sensor has detected the presence of bubbles (e.g. fob) in the beverage passage.

The conduit may extend from the connector to the valve. The conduit may be a first (tube-in-tube) conduit. The system may comprise a second conduit (having the same tube-in-tube structure as the first conduit) extending from the valve to the cooler. In other embodiments, the first conduit may extend from the connector the cooler. Substantially the entire portion of the beverage flow path extending from the connector to the cooler may extend within the beverage tube of a tube-in-tube conduit (with the outer tube being a coolant tube for coolant flow).

At least one of the conduits may be carried within an insulated carrier (e.g. a "python"). This may further help with maintaining the temperature of the beverage at a desired temperature. In some embodiment, each coolant may be carried within an insulated carrier.

At least one of the conduits may be received (e.g. bundled) within the insulated carrier with other conduits, cables and/or lines. Such components may be arranged such that the bundle (and the insulated carrier) has a non-circular shape. The insulated carrier may have a cross-section having first and second orthogonal dimensions, the first dimension (referred to herein as the width) being larger than the second dimension (referred to herein as the height). The insulated carrier may have an elongate cross-sectional shape. The insulated carrier may have an elliptical, obround or rectangular cross-sectional shape.

The first conduit may be received within a first insulated carrier with the coolant line (e.g. the coolant supply or return line). The first insulated carrier may further carry an electrical cable extending from the sensor (or controller) to the valve. The first insulated carrier may further carry a gas line.

The system may comprise a gas flow path along which gas (e.g. CO₂ or CO₂/nitrogen blend) flows in use from a gas supply to the beverage supply. The gas flow path may be defined by one or more gas lines (e.g. arranged in series). The gas provided to the beverage supply may move beverage along the beverage flow path (i.e. upon opening of a tap at the dispensing site).

The connector may form part of a connector assembly further including a housing that houses the connector. The connector assembly may be mounted at the downstream end of the first insulated carrier.

The housing may comprise an internal cavity. For example, the housing may comprise first and second housing portions mountable (e.g. releasably mountable) together to define the cavity therebetween. The housing may, for example, comprise ABS plastic. An insulating element may be provided within cavity, which may be formed of a thermally insulative material. For example, the thermally insulative material may be a plastic foam (e.g. a polypropylene foam). The insulating element may fill a substantial portion of the cavity (e.g. more than 50% of the volume of the cavity, or more than 70%).

The insulating element may comprise a recess in which the connector is received. The recess may be configured to form an interference fit with the connector (e.g. allowing insertion and removal of the connector by hand, and retaining the connector in position without further fixing). The insulating element may substantially surround the connector.

The connector housing may comprise an opening through which the first insulated carrier enters the housing. The opening may have a non-circular shape. The opening may two orthogonal dimensions of different size. For example, the opening may have a height and a width that is greater than the height. The opening may have e.g. an elliptical, obround or rectangular shape. The connector assembly may be configured such that, in use, the width is arranged horizontally. The opening may be sized and shaped for close fit with the first insulated carrier.

Thus, the opening in generally configured for receipt of an insulated having a non-circular cross-sectional shape (i.e. of the type already described above). As may be appreciated, such a shape will have one dimension about which it more readily bends than another axis (i.e. being the longer dimension). Orienting the opening as described above can make it easier to manoeuvre the connector assembly onto a beverage supply (such as a keg) because it ensures the insulated carrier more readily bends in a direction that allows vertical movement of the connector assembly.

The connector assembly may comprise a sealing arrangement for sealing about the first insulated carrier when received in the opening. This may help to prevent heat from entering the interior of the housing (i.e. so as to maintain the temperature of the beverage within the housing). The sealing arrangement may comprise one or more clamping ribs extending about an interior of the housing proximate to the opening. Each clamping rib may engage the outer surface of the first insulated carrier so as to form a seal therebetween. The clamping ribs may also help to retain the end of the insulated carrier within the connector assembly.

The reset actuator may form part of the connector assembly. For example, the reset actuator may be mounted to the housing (e.g. so as to be accessible externally of the housing). The reset actuator may be provided at an opposite end of the housing to the opening in the housing. Providing a reset actuator with an indicator, as discussed above, reduces the number of openings that must be provided in the housing. Likewise, providing the reset actuator (and indicator) on the connector assembly as opposed to, for example, on a separate control panel, makes resetting much faster for an operator (because a user must interact with the connector to change the beverage supply anyway) and also reduces the possibility of an operator mistakenly replacing the wrong beverage supply.

The connector assembly may comprise a gas line connector configured to fluidly connect the ends of two gas lines (e.g. arranged in series). Thus, the gas line connector may comprise two opposite ends, each configured for connecting to a gas line. At least one of the ends may be configured for releasable connection to a gas line. The gas line connector may be mounted to the housing such that the at least one end (e.g. the releasable connection) is exposed externally of the housing. This may permit releasable connection of a gas line extending from the connector assembly to the beverage supply. Such releasable connection is beneficial because such a gas line may be susceptible to damage (e.g. due to repeated connection/disconnection to beverage supplies) and providing such a releasable connection means that the gas line can be easily replaced.

The gas line held within the insulated carrier may be connected to the end of the gas line connector that is internal to the housing.

The valve may similarly be housed within an enclosure (referred to herein as a component enclosure). The component enclosure may comprise a housing which may be formed of first and second housing portions releasable connectable to define a cavity therebetween. An insulative element may be received in the cavity. The insulative element may define a recess in which the valve is received.

Thus, each of the sensor and the valve may be housed within an insulative element such that heat transfer to beverage flowing through these components may be reduced. Likewise, beverage transported between these components may be surrounded by coolant (and optionally insulation) which may ensure that heat is not transferred to this beverage. Accordingly, substantially the entire beverage flow path may be surrounded by coolant and/or insulation. This ensures that the entire beverage flow path can be kept at a desirable temperature such that there is no point along the beverage flow path that microorganism growth can occur.

In a second aspect not according to the invention, there is disclosed a connector assembly for connecting a beverage flow path to a beverage supply, the connector assembly comprising:
a housing defining an internal cavity;
an insulating element received in the cavity, the insulating element defining one or more recesses;
a connector for releasable connection to a beverage supply, the connector received in a recess of the insulating element and comprising a beverage passage for flow of beverage from the beverage supply;
a sensor received in a recess of the insulating element and comprising two spaced probes arranged so as to be in contact with beverage flowing through the beverage passage in use to measure a parameter of the beverage.

Providing the connector within a recess of the insulative element ensures that beverage flowing through the beverage passage can be maintained at a desired temperature (e.g. below that at which microorganism growth may occur). At the same time, providing the sensor probes at this location means that microorganism growth does not occur on the probes (thereby fouling the probes and causing erroneous measurements). The provision of the sensor and connector together in a housing helps to prevent damage to the sensor through use of the connector (e.g. during connection/disconnection of the connector to a beverage supply).

It has also been found that the provision of a housing (which is inevitably larger than the connector alone) provides a greater surface for a user to grip. This facilitates connection/disconnection of the connector to/from a beverage supply.

Optional features of the second aspect will now be set out. These are applicable singly or in any combination with any aspect.

The connector assembly of the second aspect may be as otherwise described above with respect to the first aspect (i.e. may comprise one or more features of the connector assembly described above with respect to the first aspect).

The housing may comprise first and second housing portions mountable (e.g. releasably mountable) together to define the cavity therebetween. The housing may, for example, comprise ABS plastic. The insulating element may be formed of a thermally insulative material such as e.g. a plastic foam (e.g. a polypropylene foam). The insulating element may fill a substantial portion of the cavity (e.g. more than 50% of the volume of the cavity, or more than 70%).

The recess in which the connector is received may be configured to form an interference fit with the connector (e.g. allowing insertion and removal of the connector by hand, and retaining the connector in position without further fixing). The insulating element may substantially surround the connector.

The connector housing may comprise an opening for receipt of an insulated carrier (such as an insulated carrier as described above with respect to the first aspect). The opening may have a non-circular shape. The opening may two orthogonal dimensions of different size. For example, the opening may have a height and a width that is greater than the height. The opening may be elongate. The opening may have e.g. an elliptical, obround or rectangular shape. The connector assembly may be configured such that, in use (e.g. connected on top of a beverage supply), the width is arranged horizontally. The opening may be sized and shaped for close fit with an insulated carrier.

The connector assembly may comprise a sealing arrangement for sealing about an insulated carrier when received in the opening. This may help to prevent heat from entering the interior of the housing (i.e. so as to maintain the temperature of the beverage within the housing). The sealing arrangement may comprise one or more clamping ribs extending about an interior of the housing proximate to the opening. Each clamping rib may engage the outer surface of an insulated carrier received in the opening so as to form a seal therebetween. The clamping ribs may also help to retain the end of the insulated carrier within the connector assembly.

The connector assembly may comprise a controller. The sensor may be operatively connected to the controller. Components of the sensor and the controller may be provided on a printed circuit board (PCB) housed within the housing.

The connector assembly may comprise an actuator (e.g. a push button). The actuator may be a reset actuator as described above with respect to the first aspect. The actuator may comprise an indicator. For example, in the case of a push button, the button may comprise a light (such as an LED).

For example, the actuator may be mounted to the housing (e.g. so as to be accessible externally of the housing). The actuator may be provided at an opposite end of the housing to the opening in the housing. The actuator may be mounted to the PCB.

The connector assembly may comprise a gas line connector configured to fluidly connect the opposed ends of two gas lines. Thus, the gas line connector may comprise two opposite ends, each configured for connecting to a gas line. At least one of the ends may be configured for releasable connection to a gas line. The gas line connector may be mounted to the housing such that the at least one end (e.g. the releasable connection) is exposed externally of the housing. This may permit releasable connection of a gas line extending from the connector assembly to the beverage supply.

### Brief Summary of the Figures

Embodiments will now be discussed with reference to the accompanying figures in which:
Figure 1 is a schematic view of a beverage dispense system;
Figure 2 is a section view of a tube-in-tube conduit of the beverage dispense system;
Figure 3 is a schematic view of a component enclosure of the beverage dispense system;
Figure 4 is a section view of an insulated carrier of the beverage dispense system;
Figures 5A and 5B are perspective and exploded views respectively of a connector assembly of the beverage dispense system; and
Figures 6A and 6B are perspective and section views respectively of a connector of the beverage dispense system.

### Detailed Description

Aspects and embodiments will now be discussed with reference to the accompanying figures. Further aspects and embodiments will be apparent to those skilled in the art.

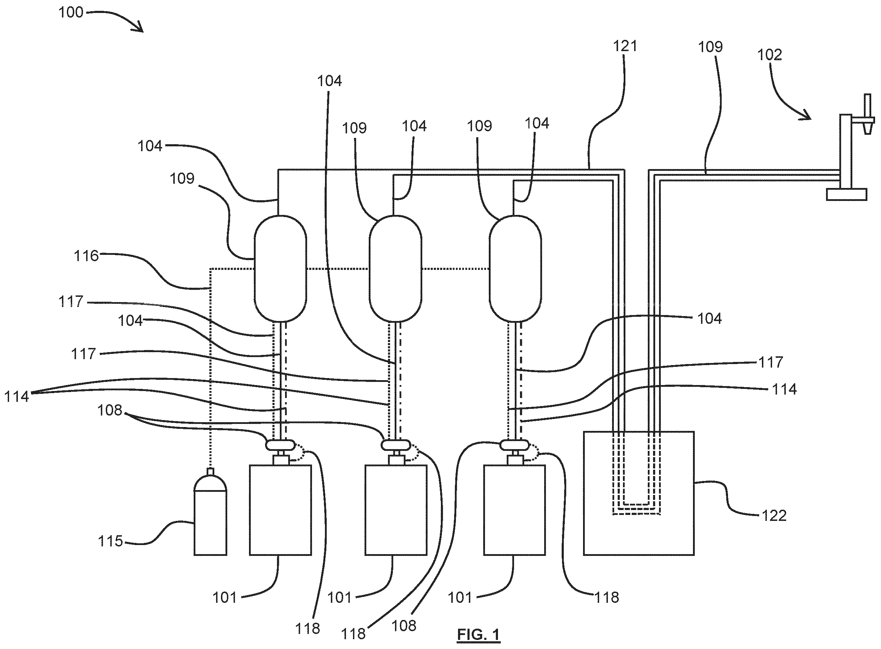

Figure 1 illustrates a beverage dispense system 100 including a beverage supply in the form of form of three kegs 101, each containing a beverage (such as e.g. beer). The kegs 101 are connected to a dispensing site 102 (e.g. in the form of a plurality of dispensing taps) by a beverage flow path along which beverage flows in operation (for example, when one of the taps is opened by an operator).

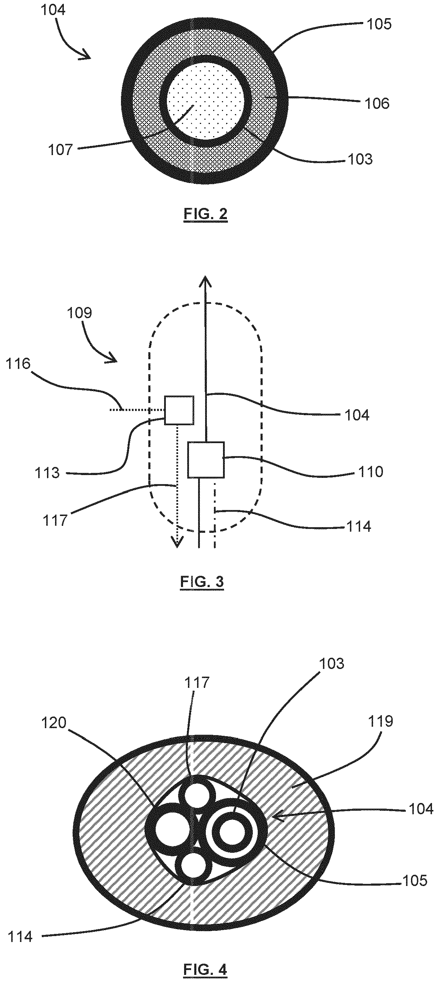

The beverage flows along the beverage flow path, between components of the system 100, within beverage tubes 103 (e.g. flexible tubes). A section of such a beverage tube 103 is shown in Figure 2. As is apparent from this figure, each beverage tube 103 forms part of a tube-in-tube arrangement 104. In particular each beverage tube 103 extends centrally within the internal passage of a corresponding coolant tube 105 (e.g. a flexible tube). In this way, each beverage tube 103 is fully surrounded (i.e. circumferentially) by an annular volume of coolant 106 (e.g. cooled water) flowing within the coolant tube 105. In this way, the coolant is able to maintain the temperature of the beverage 107 flowing within the beverage tube 103 at a desired temperature. This helps to prevent microorganism growth within and along the beverage tubes 103 (which can spoil the beverage 107 flowing through the beverage tubes 103 and beverage stored in kegs 101). For brevity, the combination of a beverage tube 103 and coolant tube 105 (i.e. in a tube-in-tube arrangement as described above) will be referred to below as a "conduit 104".

Returning to Figure 1, each keg 101 is connected to an upstream end of the beverage flow path by a connector assembly 108 (which will be described in more detail further below). Each connector assembly 108 provides for releasable connection to a corresponding keg 101. A conduit 104 extends from each connector assembly 108 to a component enclosure 109 (which is wall mounted).

Figure 3 schematically illustrates the internal assembly of such a component enclosure 109. The component enclosure 109 houses a valve 110 (e.g. a solenoid valve) configured to obstruct a portion of the beverage flow path extending through the component enclosure 109 (so as to prevent flow of beverage past the valve), for example within a conduit 104 as described above. Closure of the valve 110 prevents beverage from passing beyond the valve 110 and thus from being dispensed at the dispense site 102.

Although not illustrated, each connector assembly 108 houses a bubble sensor configured to detect the presence of bubbles in the beverage passing through the connector assembly 108. Each bubble sensor is configured to generate a signal when it detects a level of bubbles that indicates the presence of fob (or the imminent presence of fob) in the beverage. This signal is communicated to the corresponding valve 110 (located downstream of the bubble sensor) by way of an electrical cable 114 (i.e. for transmitting electrical signals). In response to receipt of the signal, the valve 110 blocks the beverage flow path, preventing supply of beverage to the dispense site 102 (and thus, for example, the further progress of fob along the beverage flow path).

Further component housed in each component enclosure 109 is a gas exchange component (GEC) 113, also known as a secondary reducing valve (SRV).

The GEC 113 is connected to a gas supply (in the form of a gas cylinder 115, shown in Figure 1) by a primary gas line 116. The gas suppled from the gas cylinder 115 may, for example, be CO₂. The GEC is configured to draw off a portion of the gas from the primary gas line 116 and to reduce the pressure of the gas, for example to a pressure that is suited to the beverage stored in the corresponding keg 101 (i.e. the keg 101 supplying beverage to the same component enclosure 109). Reduced pressure gas is transported from the GEC 113 to the connector assembly 108 in a secondary gas line 117. This reduced pressure gas is ultimately provided to a respective keg 101 by way of a tertiary gas line 118 extending from the corresponding connector assembly 108 to the keg 101 (as shown in Figure 1). The gas supplied to each keg 101 helps to move beverage from the keg 101 (i.e. when a tap at the dispensing site 102 is opened).

Each of the secondary gas line 117, conduit 104 and electrical cable 114 (of each connector assembly 108 / component enclosure 109 pair) are bundled together as shown in Figure 4. In particular, they are held within an insulated carrier 119 (also known as a "python"), which further helps to maintain the temperature of beverage in the beverage tube 103 at a desired temperature (i.e. at a temperature below which microorganism growth occurs). Also bundled within the insulated carrier 119 is a coolant return line 120. Although not shown in the schematic of Figure 1, this coolant return line 120 returns coolant from the respective connector assembly 108, received from the annular passage of the coolant tube 105, to a cooler 122 (discussed further below). As may be appreciated, in other embodiments, the coolant return line may instead be a supply line.

As should be apparent from Figure 4, the four lines/cables/conduits 104, 114, 117, 120 are arranged in a diamond configuration. This results in the insulated carrier 119 having an external shape that is elliptical.

Returning again to Figure 1, each component enclosure 109 is fluidly connected to a cooler 122 by three conduits 104 (each carrying beverage from one of the kegs 101). These three conduits 104 are bundled together within an insulated carrier. Also bundled with the three conduits 104 are portions of the coolant return lines 120 (discussed above with respect to Figure 4), which supply coolant from the cooler 122 (to each of the connector assemblies).

The cooler 122 may be in the form of an ice bank cooler. The beverage supplied from the three kegs 101 may pass through the cooler 122 (as illustrated) or may bypass the cooler 122. In either case, the cooler 122 is configured to cool and supply the coolant that flows through the cooling conduits 105 (surrounding the beverage tubes 103).

The cooler 122 is fluidly connected to three further conduits 104 that transport the beverage to the dispensing site 102. Cooling lines (not shown) are also provided to supply coolant to each of these conduits 104. Again, each of these conduits/lines are bundled in one or more insulated carriers. Thus, for every portion of the beverage flow path that is between two components of the system 100 (and that would thus otherwise be exposed to the external environment) the beverage flow path is held within an insulated carrier. Further, insulation is provided within the component enclosures 109 and connector assemblies 103, which surround the conduits 104 (i.e. effectively performing the function of the insulated carriers 119 provided between components). This ensures that the entire beverage flow path can be maintained at a temperature below which microorganism growth can occur.

Moreover, the beverage flow path, for substantially its entire length, is circumferentially surrounded by coolant (flowing within one of the coolant tubes 105). Again, this ensures that the entire beverage flow path can be maintained at or below a desired temperature.

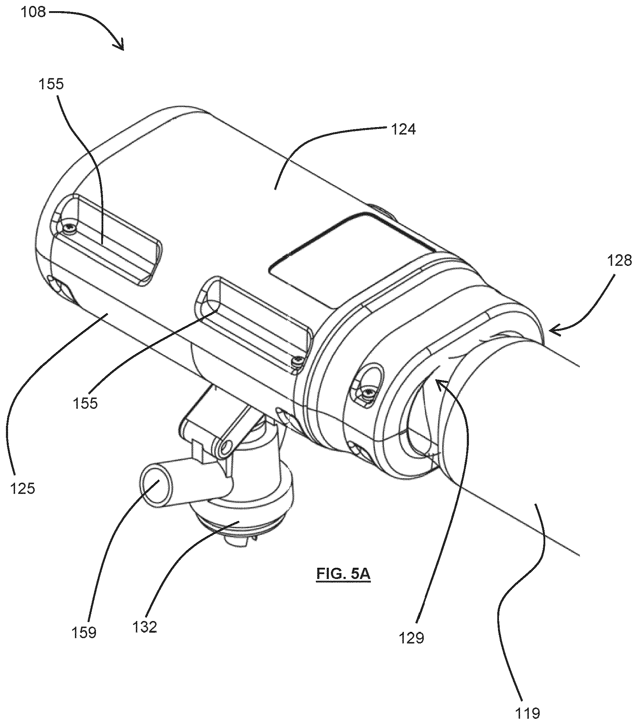

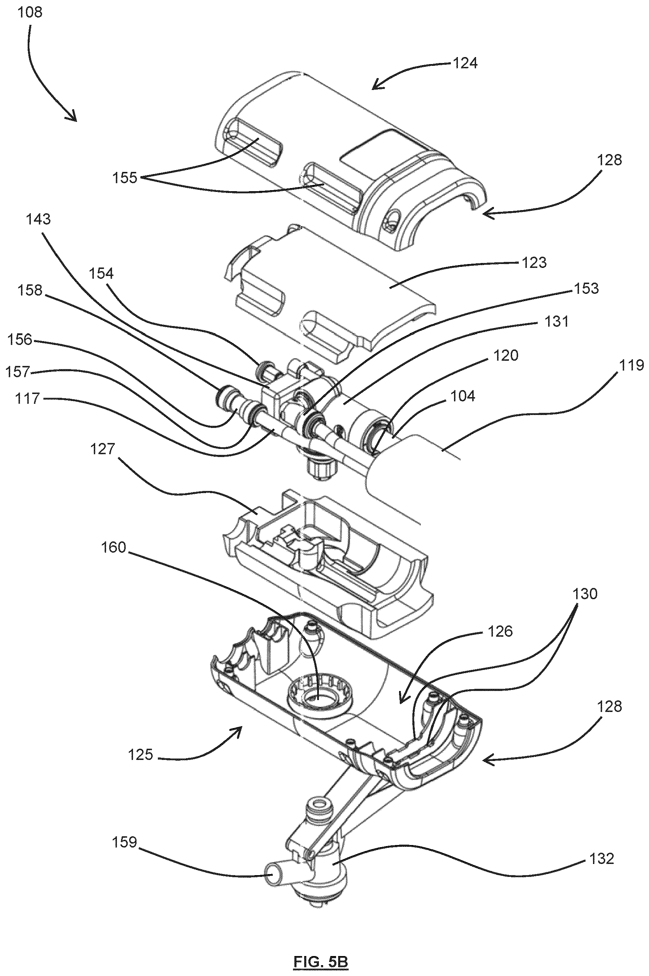

Figures 5A and 5B show a connector assembly 108 in more detail. The connector assembly 108 comprises a housing formed of upper 124 and lower 125 housing portions that, when brought together, form an internal space 126. An insulating element in the form of an insulating insert, formed of upper 123 and lower 127 insert portions, is provided within the internal space 126. The upper 123 and lower 127 portions of the insert define a plurality of recesses in which various components held by the connector assembly 108 are received.

The upper 124 and lower 125 housing portions are secured together with screws (not shown). The upper housing portion 124 includes external recesses 155 arranged in two pairs spaced laterally across the upper housing portion 124 from one another. The recesses 155 are arranged so as to allow a user to more easily grip the upper housing portion 124, which aids in disconnecting the connector assembly 108 from (and reconnecting the connector assembly 108 to) a keg 101.

A first end 128 of the connector assembly 108 comprises an obround (i.e. flattened circle) shaped opening 129. This opening 129 receives an upstream end of the insulated carrier 119 (along with bundled lines/conduits/cables) shown in Figure 4. The obround shape of the opening 129 helps to clamp the insulated carrier 119 between the two housing portions 124, 125 which provides a secure, sealed connection. This sealed connection, again, helps to maintain the temperature of the beverage (by preventing heat from the external environment entering the internal space 126). This shape also means that the insulated carrier 119 is oriented in a manner where it most readily bends in a direction that allows vertical movement towards and away from a keg 101 (so as to allow easier disconnection/connection).

To further improve the seal between the connector assembly 108 and an insulated carrier 119 (and to grip the carrier 119), the housing 124, 125 includes mounting ribs 130, which project inwardly from an internal surface of the housing 124, 125. The mounting ribs 130 extend circumferentially about the internal space 126, so as to extend about an insulated carrier 119 received through the opening 129.

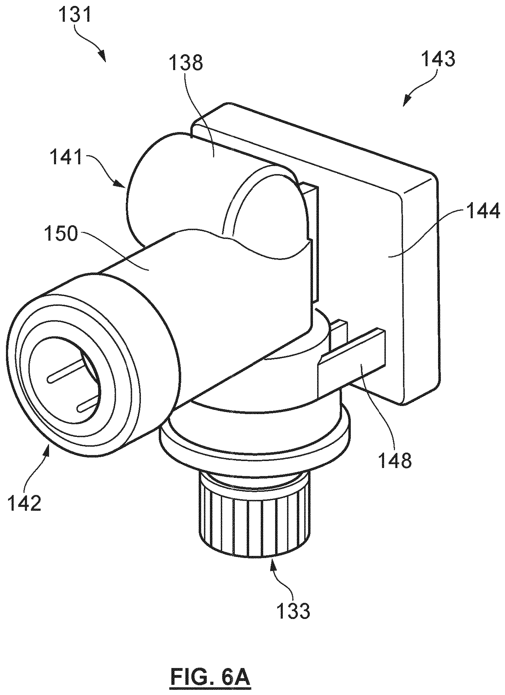

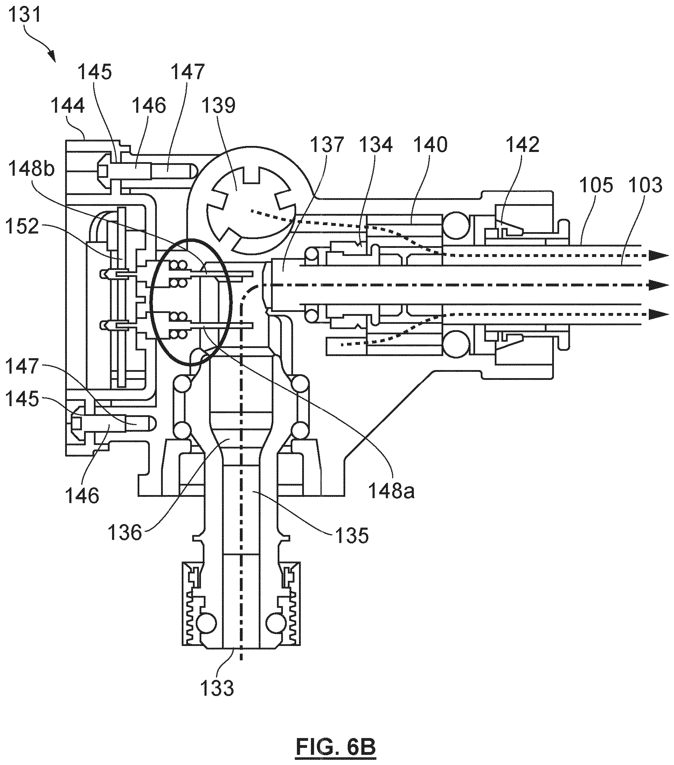

The connector assembly 108 houses a connector 131, which his shown in more detail in Figures 6A and 6B. The connector 131 is configured to releasably connect an outlet of a keg coupler 132 (see Figures 5A and 5B) to the inlet of a beverage tube. The connector 131 also provides means for connecting a coolant return line 120 to the coolant tube 105 surrounding the beverage tube 103. Thus, in general, the connector 131 includes two flow paths. A first flow path (represented by the dashed-dotted line) is for beverage and extends from a beverage inlet 133 to a beverage outlet 134. The beverage inlet 133 is releasably connectable to the keg coupler 132 (through a lower opening 160 in the lower housing portion 125) and the beverage outlet 134 is releasably connectable to a beverage tube 103.

A second flow path (represented by the dotted lines) is for coolant and extends from a coolant inlet 141 to a coolant outlet 142. The coolant inlet 141 is releasably connectable to the coolant return line 120 and the coolant outlet 142 is releasably connectable to a coolant tube 105.

As is apparent from Figure 6B in particular, the first flow path is defined within a beverage passage 135 that has first 136 and second 137 portions arranged perpendicularly to one another. The upstream portion 136 of the beverage passage 135 is adjacent to the beverage inlet 133 and the downstream portion 137 of the beverage passage 135 is adjacent to the beverage outlet 134.

The second flow path is defined within a coolant passage 138 that also has first 139 and second 140 portions arranged perpendicularly to one another. The second portion 140 of the coolant passage 138 extends circumferentially about the downstream portion 137 of the beverage passage 135 and the beverage outlet 134. The first portion 139 of the coolant passage 138 (which extends laterally in use) also extends perpendicularly to the upstream portion 136 of the beverage passage 135 (which extends vertically in use).

Also shown in Figures 6A and 6B is a bubble sensor 143 mounted to the connector 131. The bubble sensor 143 is configured to detect the presence of (and the level of) bubbles within the beverage flowing along the beverage passage 135. The bubble sensor 143 is mounted to the connector 131 by way of a sensor mount 144 in the form of a tray in which a printed circuit board (PCB) 152 of the bubble sensor 143 is received. The sensor mount 144 includes mounting portions in the form of holes 145 for receipt of screws 146 (that engage with threaded bores 147 of the connector 131).

The bubble sensor 143 is configured to detect bubbles by measuring an electrical parameter (such as conductivity or capacitance) of the beverage. To allow the bubble sensor 143 to be able to do this, the bubble sensor 143 includes upstream 148a and downstream 148b elongate (pin-like) probes that are spaced apart from one another (and are insulated from one another). The probes 148a, 148b extend through a portion 149 of an external wall 150 of the connector 131 that is adjacent to (and partly defines) the upstream portion 136 of the beverage passage 135. In this way, the probes 148a, 148b do not pass through the coolant (i.e. they pass directly into the beverage passage 135). This prevents possible erroneous measurements that could otherwise occur due to contact with coolant.

The probes 148a, 148b extend generally perpendicularly to the upstream portion 136 of the beverage passage 135 and are spaced from one another in the direction of the upstream portion 136. The probes 146 extend into the beverage passage 135 at a bend between the two portions 136, 137. Beverage flowing around the bend (and bubbles therein) will first come into contact with the upstream probe 148a before subsequently coming into contact with the downstream probe 148b. To ensure that there is no leakage of beverage from the connector 131 sealing members 151 (in the form of compressible washers) are provided for sealing between each probe and the portion 149 of the external wall 150 through which they extend.

Returning now to Figures 5A and 5B, the connector 131 is shown for fitting into the connector assembly 108. As is apparent, an elbow fitting 153 is connected to the coolant inlet 141. A coolant supply line can then be connected to the elbow fitting 153. Also apparent from these figures is that the connector assembly 108 further includes a reset actuator in the form of a push button 154 that is directly (electrically) connected to the bubble sensor 143. When pushed, the push button 154 communicates (via the bubble sensor) with the valve 110 of the system 100 (as in Figure 3) and causes the valve 110 to re-open (i.e. essentially indicating to the system 100 that the keg has been replaced).

Conveniently, the push button 154 also includes an indicator in the form of an LED that is configured to indicate to a user the status of the valve 110. Thus, for example, when the valve 110 is closed the LED may emit a red coloured light and when the valve 110 is open the LED may emit a green coloured light. This allows a user to quickly identify whether a keg 101 requires replacement and, because the light forms part of the connector assembly 108 itself, the LED also identifies which keg 101 requires replacing (i.e. so there is less possibility of a user replacing the wrong keg 101). Although not shown, indication of the status of the system may also be provided by an indicator (e.g. LED) on each component enclosure 109.

By providing a button 154 that also acts as an indicator, fewer openings need to be provided in the housing 125. This reduces the complexity of manufacture, and also aids in insulating the beverage within the connector assembly 108 (i.e. preventing heat from entering the interior housing 125).

Further housed in the connector assembly 108 is a gas connector 156. The gas connector 156 provides releasable connection to each of a secondary gas line 117 and a tertiary gas line 118 of the system 100 (as shown in Figure 1). Secondary gas line 117 extends into the connector assembly 108 from a bundle within the insulated carrier 119 and connects with an inlet 157 of the gas connector 156 (the inlet 157 providing for releasable connection). One end of the tertiary gas line 118 connects with an outlet 158 of the gas connector 156 (the outlet 158 also providing for releasable connection) and the other end connects to a gas inlet 159 of the keg coupler 132.

In practice, it has been found that the tertiary gas line 118 is particularly susceptible to damage (given it is frequently connected/disconnected when replacing kegs). The provision of a releasable connection (on the connector assembly 108) to this tertiary gas line 118 allows for easy replacement of the tertiary gas line 118 in the event that it is damaged and is no longer usable.

For the avoidance of any doubt, any theoretical explanations provided herein are provided for the purposes of improving the understanding of a reader. The inventors do not wish to be bound by any of these theoretical explanations.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

## Claims

1. A beverage dispense system (100), the system comprising:
a beverage flow path (103) along which beverage flows in use from a beverage supply (101) to a dispense site (102);
a coolant flow path (105) along which coolant flows from a cooler;
a connector (131) at an upstream end of the beverage flow path (103) configured for releasable connection to the beverage supply (101), the connector (131) comprising a beverage passage (135) along which a portion of the beverage flow path (103) extends and a coolant passage (138) along which a portion of the coolant flow path (105) extends; and
a sensor (143) comprising two spaced probes (148a, 148b) arranged so as to be in contact with beverage flowing through the beverage passage (135) in use to measure a parameter of the beverage;
**characterised in that** the beverage dispense system (100) further comprises a flexible tube-in-tube conduit (104) extending from the connector (131), the conduit (104) comprising an inner beverage tube extending within an outer coolant tube, the beverage tube connected to the beverage passage (135) so as to define a portion of the beverage flow path (103) and the coolant tube connected to the coolant passage (138) so as to define a portion of the coolant flow path (105).

2. A beverage dispense system (100) according to claim 1 wherein the connector (131) comprises a beverage inlet (133) configured for releasable engagement with the beverage supply (101) and a beverage outlet (134) connected to the beverage tube.

3. A beverage dispense system (100) according to claim 2 wherein the connector (131) comprises a coolant inlet (141) and a coolant outlet (142), the coolant passage extending from the coolant inlet (141) to the coolant outlet (142).

4. A beverage dispense system (100) according to claim 3 wherein one of the coolant inlet (141) and coolant outlet (142) is coaxial with the beverage outlet (134).

5. A beverage dispense system (100) according to any one of the preceding claims wherein a portion of the coolant passage (138) of the connector (131) circumferentially surrounds the beverage passage (135) of the connector (131).

6. A beverage dispense system (100) according to any one of the preceding claims wherein the beverage passage (135) comprises an upstream portion (136) and a downstream portion (137) that are substantially perpendicular to one another.

7. A beverage dispense system (100) according to any one of the preceding claims wherein each probe (148a, 148b) of the sensor (143) is elongate.

8. A beverage dispense system (100) according to claim 7 wherein each probe (148a, 148b) extends through a wall portion (149) of the connector (131), the wall portion (149) being a portion of an external wall (150) of the connector (131).

9. A beverage dispense system (100) according to claim 8 wherein an internal surface of the wall portion (149) partly defines the beverage passage (135) and an external surface of the wall portion (149) is an external surface of the connector (131).

10. A beverage dispense system (100) according to claim 9, when dependent on claim 6, wherein the wall portion (149) partly defines the upstream portion (136) of the beverage passage (135).

11. A beverage dispense system (100) according to any one claims 7 to 10 wherein at least one of the probes (148a, 148b) extends so as to be substantially parallel to at least a portion of the beverage passage (135).

12. A beverage dispense system (100) according to claim 11, when dependent on claim 6, wherein the at least one probe (148a, 148b) is substantially parallel to the downstream portion (137) of the beverage passage (135).

13. A beverage dispense system (100) according to any one of the preceding claims wherein the probes (148a, 148b) extend within a bend of the beverage passage (135).

14. A beverage dispense system (100) according to any one of the preceding claims wherein the probes (148a, 148b) comprise an upstream probe (148a) and a downstream probe (148b) and wherein the probes (148a, 148b) are spaced in a direction of extension of the beverage passage (135).

15. A beverage dispense system (100) according to any one of the preceding claims wherein the sensor (143) is releasably mounted to the connector (131).

## Patentansprüche

1. Getränkeabgabesystem (100), wobei das System Folgendes umfasst:
einen Getränkeströmungspfad (103), über den bei Gebrauch ein Getränk von einer Getränkeversorgung (101) zu einer Abgabestelle (102) strömt;
einen Kühlmittelströmungspfad (105), über den ein Kühlmittel von einer Kühleinheit aus strömt;
einen Verbinder (131) an einem Stromaufwärtsende des Getränkeströmungspfads (103), der für eine lösbare Verbindung mit der Getränkeversorgung (101) ausgelegt ist, wobei der Verbinder (131) einen Getränkedurchlass (135), über den sich ein Abschnitt des Getränkeströmungspfads (103) erstreckt, und einen Kühlmitteldurchlass (138), über den sich ein Abschnitt des Kühlmittelströmungspfads (105) erstreckt, umfasst; und
einen Sensor (143), der zwei voneinander beabstandete Sonden (148a, 148b) umfasst, die angeordnet sind, um bei Gebrauch mit einem Getränk, das durch den Getränkedurchlass (135) strömt, in Kontakt zu sein, um einen Parameter des Getränks zu messen;
**dadurch gekennzeichnet, dass** das Getränkeabgabesystem (100) ferner eine flexible Schlauch-im-Schlauch-Leitung (104) umfasst, die sich von dem Verbinder (131) weg erstreckt, wobei die Leitung (104) einen inneren Getränkeschlauch umfasst, der sich innerhalb eines äußeren Kühlmittelschlauchs erstreckt, wobei der Getränkeschlauch mit dem Getränkedurchlass (135) verbunden ist, um einen Abschnitt des Getränkeströmungspfads (103) zu definieren, und der Kühlmittelschlauch mit dem Kühlmitteldurchlass (138) verbunden ist, um einen Abschnitt des Kühlmittelströmungspfads (105) zu definieren.

2. Getränkeabgabesystem (100) nach Anspruch 1, wobei der Verbinder (131) einen Getränkeeinlass (133), der zum lösbaren In-Eingriff-bringen mit der Getränkeversorgung (101) ausgelegt ist, und einen Getränkeauslass (134), der mit dem Getränkeschlauch verbunden ist, umfasst.

3. Getränkeabgabesystem (100) nach Anspruch 2, wobei der Verbinder (131) einen Kühlmitteleinlass (141) und einen Kühlmittelauslass (142) umfasst, wobei der Kühlmitteldurchlass sich von dem Kühlmitteleinlass (141) bis zu dem Kühlmittelauslass (142) erstreckt.

4. Getränkeabgabesystem (100) nach Anspruch 3, wobei einer aus dem Kühlmitteleinlass (141) und dem Kühlmittelauslass (142) mit dem Getränkeauslass (134) koaxial ist.

5. Getränkeabgabesystem (100) nach einem der vorangegangenen Ansprüche, wobei ein Abschnitt des Kühlmitteldurchlasses (138) des Verbinders (131) den Getränkedurchlass (135) des Verbinders (131) umfangsmäßig umgibt.

6. Getränkeabgabesystem (100) nach einem der vorangegangenen Ansprüche, wobei der Getränkedurchlass (135) einen Stromaufwärtsabschnitt (136) und einen Stromabwärtsabschnitt (137) umfasst, die im Wesentlichen senkrecht aufeinander stehen.

7. Getränkeabgabesystem (100) nach einem der vorangegangenen Ansprüche, wobei jede Sonde (148a, 148b) des Sensors (143) länglich ist.

8. Getränkeabgabesystem (100) nach Anspruch 7, wobei jede Sonde (148a, 148b) sich durch einen Wandabschnitt (149) des Verbinders (131) hindurch erstreckt, wobei der Wandabschnitt (149) ein Abschnitt einer Außenwand (150) des Verbinders (131) ist.

9. Getränkeabgabesystem (100) nach Anspruch 8, wobei eine Innenfläche des Wandabschnitts (149) teilweise den Getränkedurchlass (135) definiert und eine Außenfläche des Wandabschnitts (149) eine Außenfläche des Verbinders (131) ist.

10. Getränkeabgabesystem (100) nach Anspruch 9 in Abhängigkeit von Anspruch 6, wobei der Wandabschnitt (149) teilweise den Stromaufwärtsabschnitt (136) des Getränkedurchlasses (135) definiert.

11. Getränkeabgabesystem (100) nach einem der Ansprüche 7 bis 10, wobei zumindest eine der Sonden (148a, 148b) sich derart erstreckt, dass sie im Wesentlichen parallel zu zumindest einem Abschnitt des Getränkedurchlasses (135) ist.

12. Getränkeabgabesystem (100) nach Anspruch 11 in Abhängigkeit von Anspruch 6, wobei die zumindest eine Sonde (148a, 148b) im Wesentlichen parallel zu dem Stromabwärtsabschnitt (137) des Getränkedurchlasses (135) ist.

13. Getränkeabgabesystem (100) nach einem der vorangegangenen Ansprüche, wobei die Sonden (148a, 148b) sich innerhalb einer Biegung des Getränkedurchlasses (135) erstrecken.

14. Getränkeabgabesystem (100) nach einem der vorangegangenen Ansprüche, wobei die Sonden (148a, 148b) eine Stromaufwärtssonde (148a) und eine Stromabwärtssonde (148b) umfassen und wobei die Sonden (148a, 148b) in einer Erstreckungsrichtung des Getränkedurchlasses (135) voneinander beabstandet sind.

15. Getränkeabgabesystem (100) nach einem der vorangegangenen Ansprüche, wobei der Sensor (143) lösbar an dem Verbinder (131) angebracht ist.

## Revendications

1. Système de distribution de boisson (100), le système comprenant :
un trajet d'écoulement de boisson (103) le long duquel la boisson s'écoule en cours d'utilisation à partir d'une alimentation en boisson (101) vers un site de distribution (102) ;
un trajet d'écoulement de liquide de refroidissement (105) le long duquel du liquide de refroidissement s'écoule à partir d'un refroidisseur ;
un connecteur (131) à une extrémité amont du trajet d'écoulement de boisson (103) configuré pour une connexion libérable à l'alimentation en boisson (101), le connecteur (131) comprenant un passage de boisson (135) le long duquel une partie du trajet d'écoulement de boisson (103) s'étend et un passage de liquide de refroidissement (138) le long duquel une partie du trajet d'écoulement de liquide de refroidissement (105) s'étend ; et
un capteur (143) comprenant deux sondes espacées (148a, 148b) agencées de manière à être en contact avec la boisson s'écoulant à travers le passage de boisson (135) en utilisation pour mesurer un paramètre de la boisson ;
**caractérisé en ce que** le système de distribution de boisson (100) comprend en outre un conduit flexible tube dans tube (104) s'étendant à partir du connecteur (131), le conduit (104) comprenant un tube de boisson interne s'étendant à l'intérieur d'un tube de liquide de refroidissement externe, le tube de boisson étant connecté au passage de boisson (135) de manière à définir une partie du trajet d'écoulement de boisson (103) et le tube de liquide de refroidissement étant connecté au passage de liquide de refroidissement (138) de manière à définir une partie du trajet d'écoulement de liquide de refroidissement (105).

2. Système de distribution de boisson (100) selon la revendication 1, dans lequel le connecteur (131) comprend une entrée de boisson (133) configurée pour une mise en prise libérable avec l'alimentation en boisson (101) et une sortie de boisson (134) connectée au tube de boisson.

3. Système de distribution de boisson (100) selon la revendication 2, dans lequel le connecteur (131) comprend une entrée de liquide de refroidissement (141) et une sortie de liquide de refroidissement (142), le passage de liquide de refroidissement s'étendant de l'entrée de liquide de refroidissement (141) jusqu'à la sortie de liquide de refroidissement (142).

4. Système de distribution de boisson (100) selon la revendication 3, dans lequel l'une de l'entrée de liquide de refroidissement (141) et de la sortie de liquide de refroidissement (142) est coaxiale avec la sortie de boisson (134).

5. Système de distribution de boisson (100) selon l'une quelconque des revendications précédentes, dans lequel une partie du passage de liquide de refroidissement (138) du connecteur (131) entoure circonférentiellement le passage de boisson (135) du connecteur (131).

6. Système de distribution de boisson (100) selon l'une quelconque des revendications précédentes, dans lequel le passage de boisson (135) comprend une partie amont (136) et une partie aval (137) qui sont sensiblement perpendiculaires l'une à l'autre.

7. Système de distribution de boisson (100) selon l'une quelconque des revendications précédentes, dans lequel chaque sonde (148a, 148b) du capteur (143) est allongée.

8. Système de distribution de boisson (100) selon la revendication 7, dans lequel chaque sonde (148a, 148b) s'étend à travers une partie de paroi (149) du connecteur (131), la partie de paroi (149) étant une partie d'une paroi externe (150) du connecteur (131).

9. Système de distribution de boisson (100) selon la revendication 8, dans lequel une surface interne de la partie de paroi (149) définit en partie le passage de boisson (135) et une surface externe de la partie de paroi (149) est une surface externe du connecteur (131).

10. Système de distribution de boisson (100) selon la revendication 9, lorsqu'elle dépend de la revendication 6, dans lequel la partie de paroi (149) définit en partie la partie amont (136) du passage de boisson (135).

11. Système de distribution de boisson (100) selon l'une quelconque des revendications 7 à 10, dans lequel au moins une des sondes (148a, 148b) s'étend de manière à être sensiblement parallèle à au moins une partie du passage de boisson (135).

12. Système de distribution de boisson (100) selon la revendication 11, lorsqu'elle dépend de la revendication 6, dans lequel la au moins une sonde (148a, 148b) est sensiblement parallèle à la partie aval (137) du passage de boisson (135).

13. Système de distribution de boisson (100) selon l'une quelconque des revendications précédentes, dans lequel les sondes (148a, 148b) s'étendent à l'intérieur d'un coude du passage de boisson (135).

14. Système de distribution de boisson (100) selon l'une quelconque des revendications précédentes, dans lequel les sondes (148a, 148b) comprennent une sonde amont (148a) et une sonde aval (148b) et dans lequel les sondes (148a, 148b) sont espacées dans une direction d'extension du passage de boisson (135).

15. Système de distribution de boisson (100) selon l'une quelconque des revendications précédentes, dans lequel le capteur (143) est monté de manière amovible sur le connecteur (131).
